# EUROPEAN PATENT SPECIFICATION

(11) **EP 2 283 893 B1**
(45) Date of publication and mention of the grant of the patent: **01.02.2012**
(21) Application number: 10189767.6
(22) Date of filing: 02.07.2008
(51) Int. Cl.: A61M 39/08, A61M 5/14

(54) **Squeeze valve**
Quetschventil
Soupape de compression

(30) Priority: 02.07.2007 EP 07111567
(43) Date of publication of application: 16.02.2011
(62) Divisional of application: 08104615.3
(73) Proprietor: Ulrich GmbH & Co. KG, 89081 Ulm (DE)
(72) Inventor: Baecke, Martin, Dr., 06847 Dessau (DE); Walther, Heide, 07745 Jena (DE); Schwerdtfeger, Uwe, 39439 Amesdorf (DE)
(74) Representative: Hellmich, Wolfgang

(56) References cited:
- EP-A- 1 086 712
- EP-A- 1 698 812
- FR-A- 2 379 290
- US-A1- 2003 212 381
- US-A1- 2006 030 821
- US-B1- 6 488 660

## Description

The invention relates to computed tomography (CT) and magnetic resonance imaging (MRI), and more specifically to a squeeze valve, which may be used for injectors for administering contrast agents for CT and MRI. Such injectors are known from the products "ohio", "mississippi" and "missouri" sold by Ulrich GmbH & Co. KG.

CT, originally known as computed axial tomography (CAT or CT scan) and body section roentgenography, is a medical imaging method for generating a three-dimensional image of the internals of an object from a large series of two-dimensional X-ray images taken around a single axis of rotation. The word "tomography" is derived from the Greek tomos (slice) and graphein (to write). CT produces a volume of data which can be manipulated, through a process known as windowing, in order to demonstrate various structures based on their ability to block the X-ray beam. Modern CT scanners allow this volume of data to be reformatted in various planes or even as volumetric (3D) representations of structures.

CT scans rely on intravenously administered contrast agents in order to provide superior image quality.

Magnetic resonance imaging (MRI), formerly referred to as magnetic resonance tomography (MRT) is a non-invasive method used to render images of the inside of an object. It is primarily used in medical imaging to demonstrate pathological or other physiological alterations of living tissues.

As CT, MRI scanners can generate multiple two-dimensional cross-sections (slices) of tissue and three-dimensional reconstructions. Unlike CT, which uses only X-ray attenuation to generate image contrast, MRI has a long list of properties that may be used to generate image contrast which is created by using a selection of image acquisition parameters that weight signal by T1, T2 or T2*, or no relaxation time ("proton-density images"). T1-weighted and T2-weighted images do not always adequately show the anatomy or pathology. Therefore, as in CT contrast agents may be administered to delineate areas of interest.

A contrast agent may be as simple as water, taken orally, for imaging the stomach and small bowel although substances with specific magnetic properties may be used. Most commonly, a paramagnetic contrast agent (usually a gadolinium compound) is given. More recently, superparamagnetic contrast agents (e.g. iron oxide nanoparticles) have become available. Diamagnetic agents such as barium sulfate have been studied for potential use in the gastrointestinal tract, but are less frequently used.

From an injector point of view, there is no big difference between CT and MRI. However, injectors for MRI must not be disturbed by high magnetic fields in the range of 1 T and the injectors must not disturb the magnetic field of the MRI. For the latter reason, injectors for MRI are often powered by batteries or direct current (DC).

There are several injectors on the market. Injectors for CT are e.g. medrad Vistron CT, medrad EnVision CT, medrad Stellant CT, medtron Injektron CT2, medtron Injektron 82CT, medtron Injektron 82 HP, medtron Accutron CT, E-Z-EM Empower CTA, E-Z-EM Empower CT, tyco / LF CT 9000 ADV, tyco / Mallinckrodt Optistat, tyco / Liebel-Flarsheim OptiVantage DH and Nemoto Dual Shot. Injectors for MRI are for example medrad Spectris Solaris, medtron Injektron MRT, medtron Accutron MR, tyco / Mallinckrodt OPTISTAR LE and Nemoto Sonic Shot 50.

All of the above-identified injectors comprise automatically driven syringes. Medtron Accutron CT and medtron Accutron MR comprise tactile keys for manual piston movement. In some injectors the syringes are disposable. For medrad EnVistron CT reloadable syringes are available. In none of the syringe injectors it is possible to apply several injections consecutively from one contrast agent container. Some syringe injectors comprise two syringes, one for a saline solution, the other one for the contrast agent.

Injectors ulrich ohio tandem, ulrich missouri, ulrich ohio M, ulrich mississippi and Swiss Medical Care CT Exprés come with roll pumps which pump contrast agent and/or saline solution from at least two different containers into the subject. This architecture enables the application of several injections consecutively from one contrast agent container.

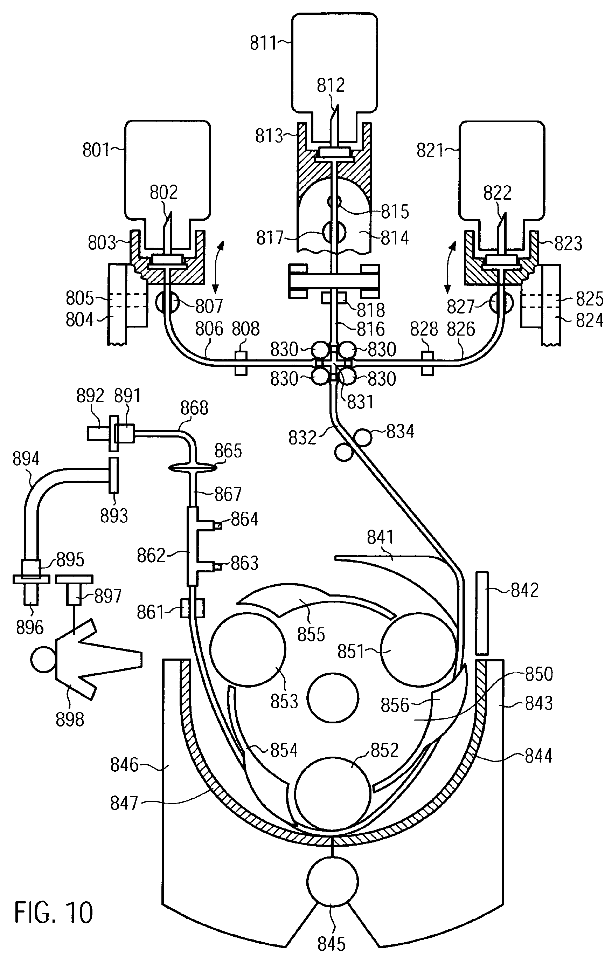

In the following we will focus on the injectors ulrich missouri and ulrich mississippi, which are identical apart from the power supply. Ulrich mississippi is battery-powered which enables MRI application. A fluid flow diagram of the injectors ulrich missouri and ulrich mississippi is shown in figure 10.

A core element of the ulrich injectors is a cross-shaped hose system for delivering contrast agent and saline solution from one of a left container 801 for a first contrast agent, a middle container 811 for saline solution and a right container 821 for a second contrast agent to the subject 898. The first and second contrast agents are often identical. Saline solution may be delivered in bottles and pouches. We use container as generic term for bottle and pouch.

The cross-shaped hose system comprises a left contrast agent hose 806, a saline solution hose 816, a right contrast agent hose 826 and a pump hose 832 which are connected by a cross connector 831. Each of the three holders for the three containers 801, 811 and 821 comprise respectively a fixed member 804, 814 and 824, and a swivel member 803, 813 and 823 which are connected by a bolt 805, 815 and 825. For removing nearly empty containers without spilling contrast agent, the swivel members 803 and 823 may be tilted forward and downward by 135° separately. For not spilling saline solution, the swivel member 813 may be tilted leftward and downward by 135°.

The left contrast agent hose 806, the saline solution hose 816 and the right contrast agent hose 826 are each fluidically connected with a piercing needle 802, 812 and 822, respectively, for piercing the rubber stopper of each container 801, 811 and 821. Ultrasonic detectors 807, 817 and 827 monitor when the respective one of the three containers 801, 811 and 821 runs empty. Stop valves 808, 818 and 828 can squeeze off each of the hoses 806, 816 and 826, respectively. Actually, plungers within the injector casing covered by a membrane press the hoses from the back against a glass cover for squeezing the hoses off. Only one of the valves 808, 818 and 828 is open at a time.

The cross connector 831 is fixed by mounting 830 to the injector casing. The pump hose 832 is fixed by mounting 834 and guidances 841 and 842 to the injector casing.

The roll pump consists of a pump wheel 850 and wings 843 and 846. The pump wheel 850 comprises three rolls 851, 852 and 853 and three guidances 854, 855 and 856, which have the form of circular arcs. Each of the wings 843 and 846 comprises a circular-arc-shaped abutment 844 and 847, respectively. During operation the pump wheel 850 turns clockwise, whereas the rolls 851, 852 and 853 turn counterclockwise. At least one of the rolls 851, 852 and 853 squeezes the pump hose 832 against one of the abutments 844 and 847 so that no fluid may flow through the squeezed portion of the pump hose 832. When the pump wheel 850 turns clockwise, squeezed portion moves towards the subject 898 thereby pumping fluid towards the subject 898.

For inserting and removing the hose system, the wings 843 and 846 may be swung outward and downward by about 45° around pivot 845.

After the roll pump, the pump hose 832 passes through in an air trap 861. Thereafter the pump hose 832 opens up out to a pressure measurement chamber 862 which comprises two measurement membranes through which two pressure sensors 863 and 864 measure the pressure. An error is output if the absolute value of the measured pressure difference between sensors 863 and 864 exceeds a threshold. After the pressure measurement chamber 862 a particle filter 865 and finally a male Luer-Lock 891 protected by a cap 892 follow.

A subject hose 894 may be connected by a female Luer-Lock 893 to the male Luer-Lock 891. At the other end of the subject hose 894 another male Luer-Lock 895 is fixed which may be either protected by a cap 896 or being connected to a cannula 897 for discharging either a contrast agent or a saline solution into the subject 898.

The hose system is replaced after each working day or after 24 hours of operation. The subject hose 894 is replaced after each subject chance.

It is the object of this invention to provide an alternate squeeze valve.

This object is achieved by the subject matter of the independent claim.

Preferred embodiments of the invention are the subject matter of the dependent claims.

The advantage of a U-shaped pump hose is that it can be easily inserted into a roll pump.

Hook-like, tunnel-like or wall-like sections are convenient for assembling the hoses onto the frame.

Fixing the contrast agent and saline solution piercing needle to the frame further simplifies inserting and removing of the hose system at the expense of a bigger frame.

A second frame, to which the contrast agent and saline solution piercing needles are fixed, advantageously reduces the size of a shipping package compared to a single big frame, to which the contrast agent and saline solution piercing needles are fixed. The solution with a second frame constitutes an advantageous trade-off between a small frame and many handling points and a big frame with few handling points.

Any frame may constitute part of the sterile shipping packaging of the hose system, thereby advantageously saving material for the packaging.

An ultrasonic flow measuring section advantageously provides a second, redundant means for flow measurement in addition to the roll pump. Consequently, error states may more reliably be detected.

An upper and lower panel which are glued or welded to each other allow a more automated assembling of the frame. To this end, the upper panel comprises guidances, which form part of the contrast agent or saline solution conduit.

An additional membrane fixed to the upper panel advantageously allows to integrate a number of functional units like de-aeration and squeeze valves, and interfaces like a pressure measurement interface into the frame without the need of manually fiddling around with hoses during assembling of the hose system.

A swelling in the membrane above or below a valve cavity renders the membrane more robust. A swelling which extends into the valve cavity arranges for a reliably tight closing of the squeeze valve at a moderate force applied by the plunger.

A pressure measuring interface integrated into the frame allows to re-use the expensive pressure sensors. It is noted that the frame with the hose system is to be replaced each day.

An abutment advantageously ensures a reliable pressure measurement by a pressure sensor outside the frame and the hose system.

The advantage of a first connection conduit, which connects an output of the saline solution valve with an output of the contrast agent valve and connecting the pump hose to the output of the contrast agent valve reduces dead space in the conduits. For the same purpose a second connection conduit may connect the output of the contrast agent valve with an output of a second contrast agent valve.

In the following preferred embodiments of this invention are described referring to the accompanying drawings.
Fig. 1 shows a first embodiment of a first inventive hose system.
Fig. 2 shows a second embodiment of the first inventive hose system.
Fig. 3 shows a third embodiment of the first inventive hose system.
Fig. 4 shows a first embodiment of a cartridge for a second inventive hose system.
Fig. 5 shows a first embodiment of a cartridge for a third inventive hose system.
Fig. 6 shows a cross-section through an embodiment of an open squeeze valve.
Fig. 7 shows a cross-section through the embodiment of the squeeze valve of figure 6 in a closed position.
Fig. 8 shows a cross-section through a pressure interface.
Fig. 9 shows a different embodiment for merging the fluids.
Fig. 10 shows a fluid flow diagram of a conventional injector.

While the present invention is described with reference to the embodiments as illustrated in the following detailed description as well as in the drawings, it should be understood that the following detailed description as well as the drawings are not intended to limit the present invention to the particular illustrative embodiments disclosed, but rather the described illustrative embodiments merely exemplify the various aspects of the present invention, the scope of which is defined by the appended claims.

The main difference of the inventive hose systems with respect to a conventional hose system shown in figure 10 is that at least part of the hoses is mounted onto a more or less rigid frame, which may be a cartridge. For the new generation of injectors it is contemplated that additional sensors, namely an ultrasonic flow sensor 51 and an ultrasonic bubble sensor 71 are integrated into the new injectors. Consequently, it will be more complicated to insert the hose system, in particular the pump hose. The purpose of the frame is mainly to simplify inserting of the hose system and make the inserting less error-prone.

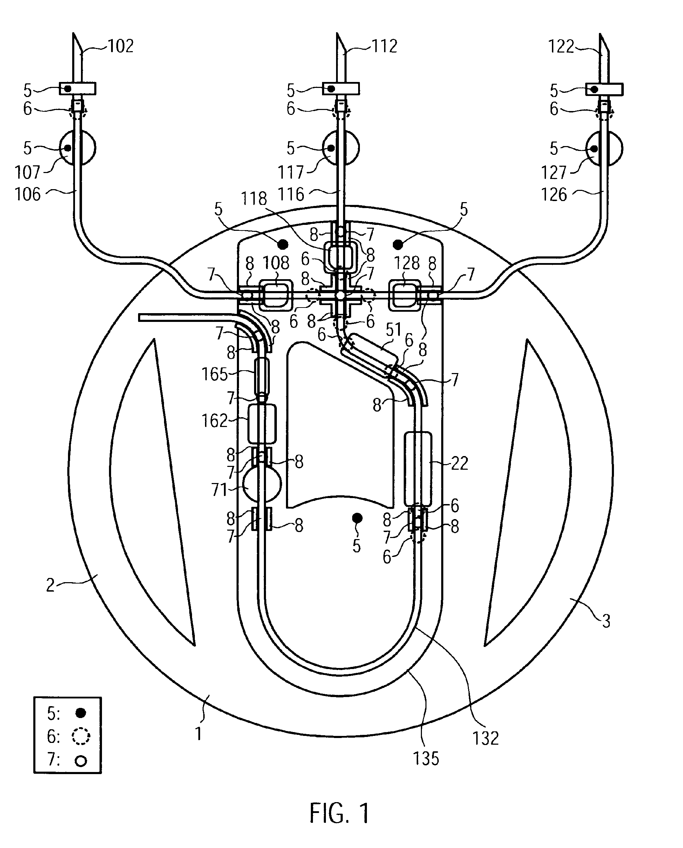

Figure 1 shows a first embodiment of a first inventive hose system. In the upper part of figure 1 piercing needles 102, 112 and 122 for a first contrast agent, saline solution and a second contrast agent, respectively, are shown. Below each of the piercing needles 102, 112 and 122 a respective ultrasonic detector 107, 117 and 127 is positioned for detecting when a respective container runs empty. Through a left contrast agent hose 106, a saline solution hose 116 and a right contrast agent hose 126 contrast agents and saline solution flow down to a injector casing 1. Before the hoses 106, 116 and 126 disembogue into pump hose 132, squeezing areas 108, 118 and 128 are provided, which operate as stop valves. During operation at least two hoses are squeezed off that only liquid through the un-squeezed hose is delivered to the subject. The plungers for squeezing the hoses maybe solenoid-operated, which is known in the art.

The pump hose 132 delivers the fluid to an ultrasonic flow sensor 51 and further to a de-aeration area 22. The U-shaped arc of the pump hose 132 is inserted into a roll pump which is not shown in figures 1 to 5. Then the pump hose 132 passes through an ultrasonic bubble sensor 71 and opens up to a pressure measurement chamber 162. Then the particle filter 165 follows which is part of the disposable hose system. Then the pump hose 132 leaves the injector casing 1. The hoses 106, 116, 126 and 132 are mounted on the lower, injector-casing-facing side of a rigid frame 135. The hoses 106, 116, 126 and 132 are fixed by hook-like, tunnel-like or wall-like sections 8 on the lower side of the frame 135. Consequently, the de-aeration area 22, the ultrasonic flow sensor 51, the ultrasonic bubble sensor 71 and the pressure measurement chamber 162 are defined from the frame 135 towards the injector casing 1.

The injector casing 1 has two handles 2 and 3.

Handling points are marked with a reference numeral 5. Handling points 5 are to be handled by an operator when inserting the hose system into the injector, more specifically the injector casing 1. There are only three handling points on frame 135. The price for the small number of handling points is a large number of frame mounting points designated by reference numerals 7 and hose mounting points designated by reference numeral 6. Frame mounting points 7 are points at which the hoses are fixed to the frame 135 when the hose system is assembled. Hose mounting points are points at which the hoses are fixed to something else than the frame 135, e. g. piercing needles 102, 112 and 122.

In total there are 9 handling points 5, 10 frame mounting points 7 and 11 hose mounting points 6.

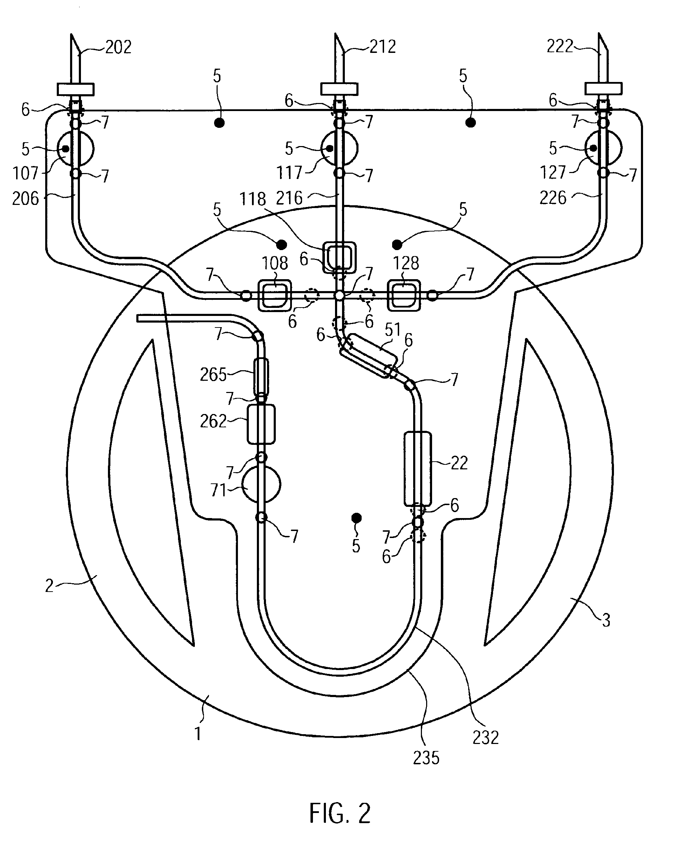

Figure 2 shows a second embodiment of the first inventive hose system. The important difference to the first embodiment shown in figure 1 is that the frame 235 is bigger. The top of the frame 235 extends up to the piercing needles 202, 212 and 222 which are fixed to the frame 235.

The second embodiment has as few as 3 handling points 5 at the expense of 16 frame mounting points 7 and again 11 hose mounting points 6. Due to the few handling points 5 the inserting and removal of a hose system according to the second embodiment is actually even simpler than the inserting and removal of a hose system according to the first embodiment. Since the frame 235 is bigger than the frame 135, the packaging of the second embodiment of the first inventive hose system will require more space. Due to 16 frame mounting points 7, assembling of the second embodiment of the first inventive hose system is likely to be more expensive. Since the piercing needles 202, 212 and 222 are fixed to the frame 235, it is not possible to swivel down any of the containers 801, 811 and 821 by 135° for a drip-free changeover of the containers 801, 811 and 821.

It should be noted that the piercing needles 202, 212 and 222 have a vertical orientation, whereas the side of the injector casing onto which the frame 235 is mounted is inclined by approximately 40° to 50° with respect to the vertical direction. Consequently, the frame 235 cannot be substantially flat, rather the frame 235 has to be kinked. The upper and lower part of the frame 235 include an angle of 40° to 50°.

The second embodiment further comprises a left contrast agent hose 206, a saline solution hose 216, a right contrast agent hose 226, a pump hose 232, a pressure measurement chamber 262 and a particle filter 265.

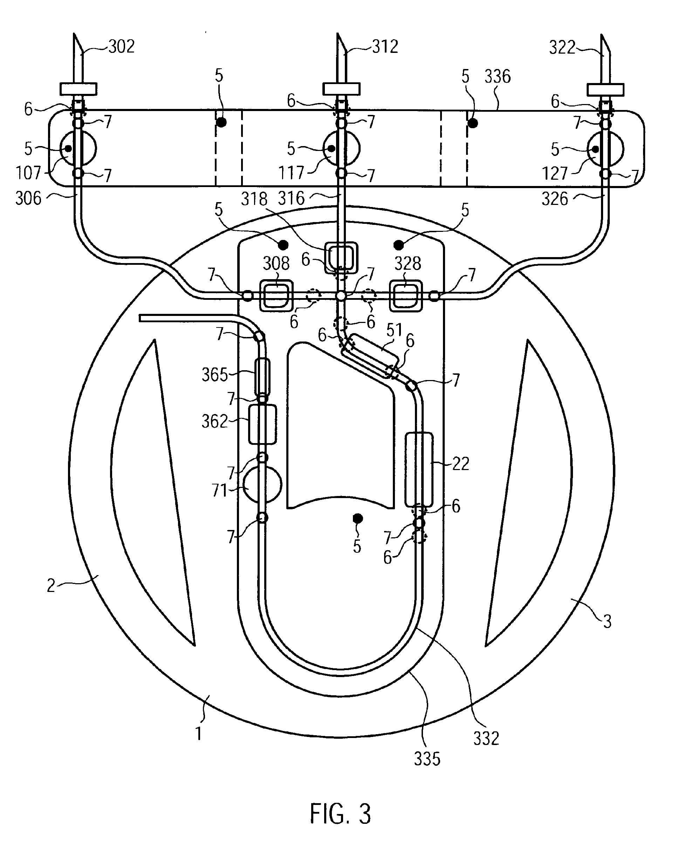

Figure 3 shows a third embodiment of the first inventive hose system, which constitutes a trade-off between the first and second embodiments of the first inventive hose system. The third embodiment comprises a lower frame 335, which is similar to frame 135, and an upper frame 336. The lower frame 335 and the upper frame 336 are connected by a left contrast agent hose 306, a saline solution hose 316 and a right contrast agent hose 326. Since all hoses are flexible, the upper frame 336 may be bent with respect to the lower frame 335, e.g. by 135° for a drip-free changeover of the containers 801, 811 and 821. Due to the fixation of the piercing needles 302, 312 and 322 to the upper frame 336, a swivel mechanism may be incorporated into the third embodiment which allows to swivel down the three containers 801, 811 and 821 together, but not to swivel down each of the three containers 801, 811 and 821 individually. The packaging of the third embodiment will require less space than the packaging of second embodiment.

The third embodiment comprises 5 handling points 5, 16 frame mounting points 7 and 11 hose mounting points 6.

The third embodiment further comprises a pump hose 332, a pressure measurement chamber 362 and a particle filter 365.

The hook-like, tunnel-like or wall-like sections 8 for fixing hoses 206, 216, 226, 232, 306, 316, 326 and 332 are not shown in figures 2 and 3, but have similar positions as shown in figure 1.

Each of the frames 135, 235, 335 and 336 may be an injection molded or deep drawn component.

In another embodiment the frames 135, 235, 335 and 336 may be part of a sterile packaging which is necessary for shipping the inventive hose systems. In this embodiment, the frames 135, 235, 335 and 336 may be produced by deep drawing. The sterile packaging may for example be a blister pack or a hard blister pack.

In order to provide a hose system, which is more suited for automated assembling, the inventors suggest to use a cartridge for the frames 135, 235 and 335.

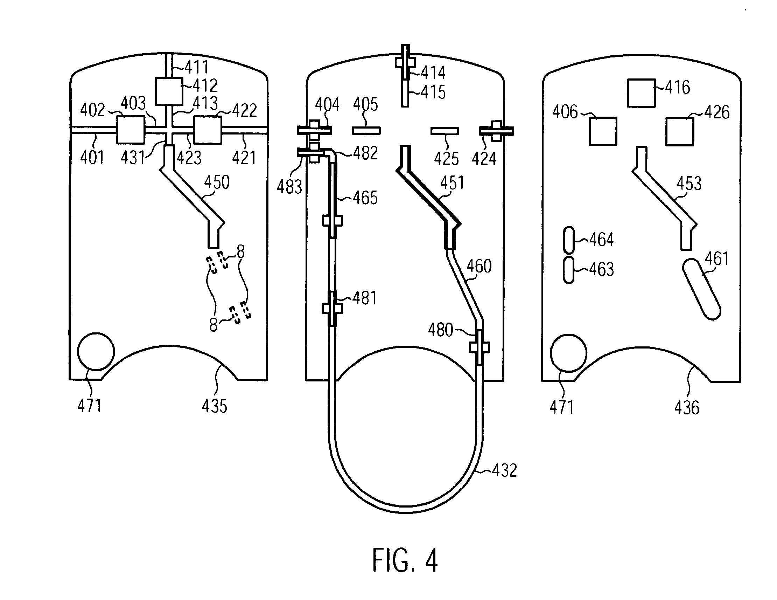

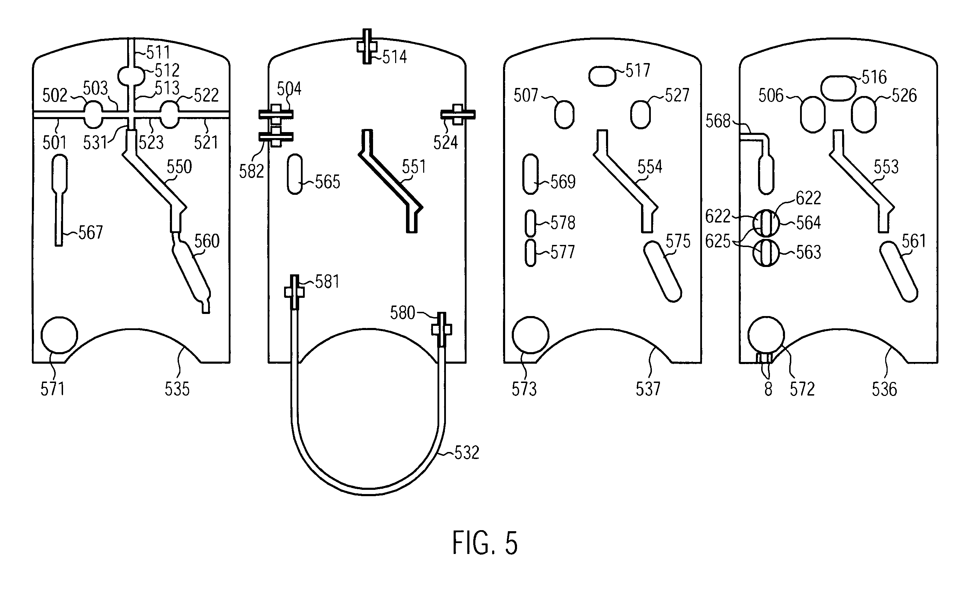

Figures 4 and 5 show first embodiments of a frame in the form of a cartridge for a second and third inventive hose system. The first embodiments shown in figures 4 and 5 are similar to the first embodiment shown in figure 1. Second embodiments of the second and third hose system having a bigger cartridge similar to frame 235 can be easily obtained by skilled persons. Third embodiments of the second and third hose system can be obtained by replacing the lower frame 335 by one of the cartridges shown in figures 4 and 5, respectively.

The cartridge for the second hose system shown in figure 4 has a sandwich-like structure. Between an upper panel 435 and a lower panel 436 inlaid members as shown in the middle of figure 4 are fixed. The upper panel 435, the lower panel 436 and the inlaid members may be mechanically connected by welding or gluing.

The upper panel 435 comprises guidances 401 and 403 which replace part of the left contrast agent hose. After the upper and lower panels 435 and 436 have been assembled, the guidances form part of conduits. In this document "conduits" is a generic expression which includes rigid tubes formed by the guidances and panels, and flexible hoses. The upper panel 435 further comprises guidances 411 and 413 which replace part of the saline solution hose. In addition, the upper panel 435 comprises guidances 421 and 423 for replacing part of the right contrast agent hose. The left contrast agent hose, the saline solution hose and the right contrast agent hose may be glued to pull relieves 404, 414 and 424, respectively, which are inlaid members in the embodiment shown in figure 4. In another embodiment the pull relieves 404, 414 and 424 may be integrated into the upper and lower panel 435 and 436 so that the left contrast agent hose, the saline solution hose and the right contrast agent hose are directly glued or welded to do the upper and lower panels 435 and 436.

Guidance 431 defined within upper panel 435 together with ultrasonic flow measuring section 451 and hose 460 replace part of the pump hose. An extra cross connector is not necessary. Hose 460 is fixed by hook-like, tunnel-like or wall-like sections 8, which protrude from the upper panel 435 to the lower panel 436.

The upper and lower panels 435 and 436 comprise cut-outs 402 and 403, which allow that hose piece 405 is squeezed off by a plunger, which is known in the art as explained above. Consequently, hose piece 405 operates like a stop valve. In a similar fashion cut-outs 412 and 416 together with hose piece 415 and cut-outs 422 and 426 together with hose piece 425 operate like two other stop valves. As mentioned above, during operation at least two hose pieces are squeezed off that only liquid through the un-squeezed hose piece is delivered to the subject.

It is worth noting that the plungers for squeezing off the hose pieces 405, 415 and 425 are mounted in the injector casing 1. When the cartridge is inserted into the injector, the lower panel 436 is facing the injector casing. Consequently, in another embodiment, the cut-outs 402, 412 and 422 may be missing in the upper panel 435, rather the respective areas of the upper panel 435 may constitute an abutment, against which the plungers squeeze those pieces 405, 415 and 425.

The pump hose 432 is fixed by pull relieves 480 and 481 to the upper and lower panels 435 and 436. The pump hose 432 may be glued or welded to the pull relieves 480 and 481. Since the pump hose is kneaded and pulled by the roll pump and the roll pump generates a pressure of up to 20 bar, the pump hose 432 may be additionally fixed to the pull relieves 480 and 481 by hose clamps and the pull relieves 480 and 481 may be bigger than the pull relieves 404, 414 and 424. Due to the pressure of up to 20 bar, hoses 462 and 482 rather than guidances integrated into the upper and lower panels 435 and 436 are suggested for delivering the fluids from the subject-sided end of the pump hose 432. Another reason for this design is that hose 462 operates as pressure measurement chamber, which is mechanically connected to the pressure transducers (not shown in figure 4) through cut-outs 463 and 464. Here, the upper panel 435 constitutes an abutment for the hose 462 and the pressure transducers. As mentioned above, two pressure transducers measure the pressure and if the absolute value of the pressure difference exceeds a threshold, an error is output.

Between hoses 462 and 482 the particle filter 465 is positioned. Since there may be a substantial pressure drop over the particle filter 465 and the pressure drop depends on the flow through the particle filter 465, the particle filter 465 has two small wings acting as pull relieves like other pull relieves 404, 414 and 424. Finally another pull relief 483 connects the short hose 482 to another hose, which is not shown in figure 4 and which delivers the fluids to a Luer-Lock and further to the subject hose (cf figure 10).

For the ultrasonic flow measuring section 451 two cut-outs 450 and 453 are provided in the upper panel 435 and the lower panel 436, respectively. The cut-outs 450 and 453 are bigger than the ultrasonic flow measuring section 451, because the ultrasonic transducers must have access to the flat surfaces terminating the measuring distance. The ultrasonic transducers are not shown in figure 4 and are fixed to the injector casing 1. The upper cut-out 450 is not necessary. The lower cut-out 453 may be split into two smaller cut-outs near the two surfaces of the ultrasonic flow measuring section 451 to be touched by the ultrasonic transducers.

The cut-out 461 provides access to the hose 460 for de-aeration purposes. The cut-outs 472 and 471 provide access to the pump hose 432 for bubble detection by an ultrasonic detector.

The hoses 460, 462 and 482 and the pump hose 432 may be fixed and guided by hook-like, tunnel-like or wall-like sections 8, which are fixed to the upper and/or lower panels 435 and 436. Such sections are also shown in figure 1.

The first embodiment of the second hose system comprises 9 handling points, 5 frame mounting points and 17 hose mounting points.

The second embodiment of the second hose system comprises 3 handling points, 11 frame mounting points and 17 hose mounting points.

The third embodiment of the second hose system comprises 5 handling points, 11 frame mounting points and 17 hose mounting points.

The handling points of the three embodiments of the second hose system are similar to the handling points of the respective embodiment of the first hose system. The number of hose mounting points is identical for the embodiments of a hose system.

Figure 5 shows a first embodiment of a cartridge for a third hose system. The main difference between the embodiments shown in figures 4 and 5 is a membrane 537, which allows a more complete integration of the cartridge and avoids the need for short hoses within the cartridge.

As the cartridge for the second hose system, the cartridge shown in figure 5 is a sandwich-like structure between an upper panel 535 and a lower panel 536. The membrane 537 is directly fixed, e. g. glued onto the lower panel 536. Onto the membrane 537 the inlaid members 504, 514, 524, 551, 565, 580, 581 and 582 are mounted e. g. by gluing. Finally, the upper panel 535 is mounted on top of the inlaid members and the membrane 537. When the cartridge is inserted into the injector, the lower panel 536 constitutes the injector-casing-near side of the cartridge.

The upper panel 535 comprises guidances 501 and 503, and a valve cavity 502 which replace part of the left contrast agent hose. The upper panel 535 further comprises guidances 511 and 513, and valve cavity 512 which replace part of the saline solution hose. In addition, the upper panel 535 comprises guidances 521 and 523, and valve cavity 522 for replacing part of the right contrast agent hose. The left contrast agent hose, the saline solution hose and the right contrast agent hose may be glued to pull relieves 504, 514 and 524, respectively. In another embodiment the pull relieves 504, 514 and 524 may be integrated into the upper and lower panel 535 and 536 so that the left contrast agent hose, the saline solution hose and the right contrast agent hose are directly glued or welded to the upper and lower panels 535 and 536.

Guidance 531 defined within upper panel 535 together with ultrasonic flow measuring section 551 and broad guidance 560 replace part of the pump hose. An extra cross connector as shown in figure 10 is not necessary. Cut-out 550 is not necessary. What has been explained above in connection with the cut-outs 450 and 453 also applies to cut-outs 553 and 554.

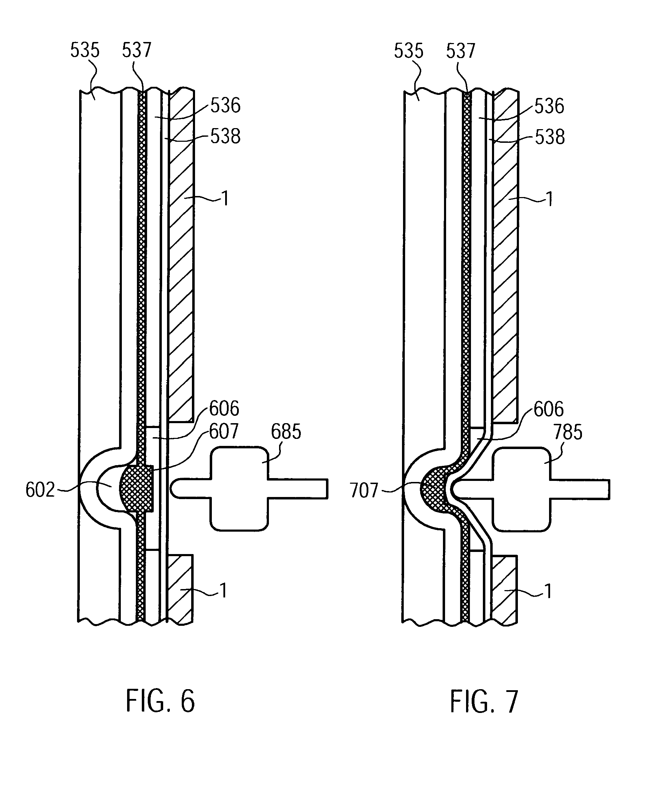

Valve cavities 502, 512 and 522 together with cut-outs 506, 516 and 526 and the respective membrane areas 507, 517 and 527 form three stop valves which will be explained in more detail in connection with figures 6 and 7. As will be explained in connection with the cross-sections in figures 6 and 7, the membrane may be thicker in the membrane areas 507, 517 and 527, which will be designated as swelling.

As in the embodiment shown in figure 4, the plungers for operating the membrane areas 507, 517 and 527 are mounted in the injector casing 1. In the embodiment shown in figure 5, the valve cavities 502, 512 and 522 constitute an abutment, against which the plungers squeeze the respective membrane areas 507, 517 and 527.

The pump hose 532 is fixed by pull relief 580 and 581 to the upper and lower panels 535 and 536. The pump hose 532 may be glued or welded to the pull relieves 580 and 581. Again, the pump hose 532 may be additionally fixed to the pull relieves 580 and 581 by hose clamps and the pull relieves 580 and 581 may be bigger than the pull relieves 504, 514 and 524.

The broad guidance 560 together with a cut-out 561 and the respective membrane area 575 serve de-aeration purposes. The thickness of the membrane in the membrane area 575 may be increased. Actually, a cross-section through the broad guidance 560, the cut-out 561 and the membrane area of 575 may be similar to a cross-section through a squeeze valve. The difference to a squeeze valve is that a plunger beats the membrane area of 575 repeatedly in order to make the fluid before the broad guidance 560 move repeatedly backwards and forward. Thereby, bubbles move upward into the respective container. No attempt is made to completely close the broad guidance 560.

At the subject-sided end of the pump hose 532, the cut-outs 571, 572 and 573 provide access to the pump hose 532 for bubble detection by an ultrasonic detector. Hook-like, tunnel-like or wall-like sections 8 protruding from the lower panel 536 to the upper panel 535 guide the pump hose 532 so that the pump hose 532 is reliably inserted into the ultrasonic detector. Then a guidance 567 delivers the liquid to a filter tissue 565, which constitutes a particle filter. Another guidance 568, which is formed by the lower panel 536, collects the liquid after the filter tissue 565 and delivers the liquid to another pull relief 582, which connects to another hose, which is not shown in figure 5 and which delivers the fluids to a Luer-Lock and further to the subject hose (cf figure 10).

When designing the gluing and/or welding areas between the upper panel 535 and the lower panel 536, it has to be borne in mind that the guidances 567 and 568 and the particle filter in between must stand pressures up to 20 bars. Consequently, the overlapping sections of guidances 567 and 568 and the filter tissue 565 should be long and narrow in order to keep the forces per length unit along the overlapping sections small. This may also be achieved by a comb or rib-like structure for a short and broad filter tissue. Another possibility is to move the filter tissue to the low-pressure side before the pump hose 532. A third possibility is to design the injector casing of the injector so that the upper and lower panels 535 and 536 can lean to two parts of the injector casing. In other words: the two parts of the injector casing act as abutments to the force generated by a high pressure during operation.

The upper and lower panels 535 and 536 may be glued to the two sides of the membrane 537. In addition or alternatively the membrane of 537 may comprise additional cuts-outs, which are not shown in figure 5, through which the upper and lower panels 535 and 536 may be directly glued or welded.

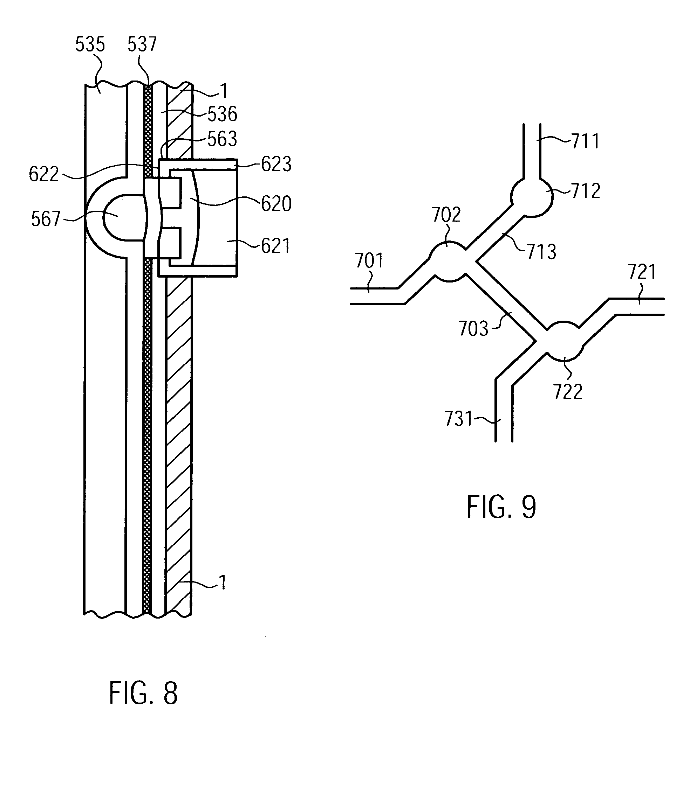

The guidance 567 together with recesses 563 and 564 and the respective swellings 577 and 578 of the membrane form two pressure interfaces equivalent to the measurement chamber. The pressure interfaces will be explained in more detail in connection with figure 8. As mentioned above, two pressure transducers measure the pressure and if the absolute value of the pressure difference exceeds a threshold, an error is output.

The first embodiment of the third hose system comprises 9 handling points, 6 frame mounting points and 10 hose mounting points.

The second embodiment of the third hose system comprises 3 handling points, 11 frame mounting points and 14 hose mounting points.

The third embodiment of the third hose system comprises 5 handling points, 11 frame mounting points and 10 hose mounting points.

The handling points of the three embodiments of the third hose system are similar to the handling points of the respective embodiment of the first hose system. The reduced numbers of hose mounting points of the three embodiments of the third hose system compared to the respective one of three embodiments of the second hose system show that the assembling of the third hose system is automatable to a great extent.

Although the pump hoses 132, 232, 332, 432, 532 have been described as U-shaped in connection with figures 1 to 5, it is clear to skilled persons, that the pump hoses are flexible and the course of a pump hose depends on the roll pump. The U shape corresponds to a pumping angle of 180°. However the pumping angle may be smaller or larger than 180°. Larger angles result in an Ω shape of the pump hose.

Figure 6 shows a cross-section through an embodiment of an open squeeze valve, e.g. one of squeeze valves 502, 512 and 522. The upper panel 535 comprises a valve cavity 602. On the right-hand side of the upper panel 535 a membrane 537 and the lower panel 536are fixed. The first membrane 537 has a swelling 607 within the valve cavity 602 and a cut-out 606. The swelling has approximately the form of the valve cavity 602 when viewed from a direction normal to the membrane 537. The cross-section in figure 6 shows that the part of the swelling extending into the valve cavity 602 may have a cross-section similar to the cross-section of the valve cavity for proper closing. The part of the swelling extending into the cut-out 606 has a rectangular cross-section.

Right of the swelling 607, the lower panel 536 has the cut-out 606 which is bigger than the valve cavity 602 and the swelling 607 viewed from a direction normal to the membrane 537.

The casing membrane 538 is fixed to the injector casing 1 to protect and in particular seal the plunger 685 and the interior of the injector casing against spilt over contrast agent and saline solution when the containers are changed and other detrimental influence.

Figure 7 shows the squeeze valve of figure 6 in a closed position. The plunger 785 has stretched the casing membrane 538 through the cut-out 606 onto the swelling 707 first membrane 537 and presses the swelling 707 into the valve cavity 602. The swelling 707 is deformed and fills the valve cavity. Consequently, any flow is stopped through the squeeze valve.

Figure 8 shows a cross-section through a pressure interface, when the cartridge of figure 5 is inserted into the injector. A pressure sensor 621 is used which has a gel pad 620 at its pressure sensing surface. In another embodiment a silicon pad could be uses instead of the gel pad, since a silicon pad is less expensive. The outer shape of the housing 623 of the pressure sensor 621 matches the form of the recess 563 so that the housing 623 of the pressure sensor 621 abuts to the abutment area 622. The housing 623 is mounted within the injector casing 1. As shown in figure 5, in the center of the recesses 563 and 564 there are oblong cuts-outs 625, which correspond to the swellings 577 and 578 of the membrane 537. As shown in figure 8, the swelling 577 and the abutment area 622 form a flat surface, after the cartridge has been assembled, provided that there is ambient pressure in guidance 567. If there is an overpressure in guidance 567, the center of the swelling 577 is bent towards the pressure sensor 621 and the gel pad 620 transmits the overpressure to the pressure sensor 621. The upper panel 535 is already shown in figure 5.

Figure 9 shows a different embodiment for merging the fluids. In particular the guidances 403, 413, 423 and 431 in the embodiments illustrated in connection with figure 4 or the guidances of 503, 513, 523 and 531 in the embodiments illustrated in connection with figure 5 may be replaced by guidances 713, 703 and 731.

The main purpose of this embodiment is to reduce the mixing of saline solution and contrast agent. A dilution of contrast agent by saline solution is less detrimental than the contamination of saline solution by contrast agent. In this regard, the embodiment shown in figure 5 is already improved by a factor of four, since the length of the conducts 503, 513 and 523 between the valve cavities 502, 512 and 522 is by a factor of four shorter than the length of the hoses 806, 816 and 826 between the stop valves 808, 818 and 828. The embodiment shown in figure 9 is further improved since there is no dead space in the guidances 703, 713 and 731, when saline solution is flowing.

The guidances 713, 703 and 731 connect the valve cavities 712, 702 and 722 in a cascade-like or meander-like manner. The result is that the saline solution provided by guidance 711 flushes the guidances 713, 703 and 731. Dead spaces for contrast agents are minimized to dead spaces with the squeeze valves corresponding to the valve cavities 712, 702 and 722. In the previous embodiments, guidances 403 and 423 as well as 503 and 513 constitute dead spaces for contrast agents, when saline solution is flowing.

When the left contrast agent is flowing, the design shown in figure 9 is still advantageous over the cross design shown in figures 4 and 5, since only guidance 713 constitutes dead space which is less than the volume of guidances 413 and 423 or 513 and 523. In this context it has to be borne in mind that the specific weight of saline solution is smaller than the specific weight of contrast agent. Therefore, if the valve corresponding to valve cavity 702 is open and the other two valves are closed, the saline solution within guidance 713 swims on top of the contrast agent and consequently does not dilute contrast agent much.

Guidances 703 and 713 constitute dead space for the right contrast agent originating from guidance 721. If comparable design parameters like guidance width and distance of valves are used, the guidances 713 and 703 comprise more dead space than the guidances 503 and 513 or 403 and 413. However, guidance 713 is not critical, since the saline solution within swims on top of the contrast agent and the dead space within guidance 703 is smaller than the dead space than the guidances 503 and 513 or 403 and 413. In addition, if a mixing of different contrast agents must be avoided, the guidances 713 and 703 can be rinsed by saline solution.

## Claims

1. A squeeze valve comprising:
an upper panel (535) comprising a valve cavity (502; 512; 522; 602) separating two parts of a conduit (501, 503; 511, 513; 521, 523);
a membrane (537) being fixed to said upper panel (535) covering said valve cavity (502; 512; 522; 602) and forming a side of said parts of said conduit (501, 503; 511, 513; 521, 523); and
a plunger (685, 785) having an open position and a closed position, said plunger (685, 785) squeezing said membrane into said valve cavity (502; 512; 522; 602) thereby separating said parts of said conduit (501, 503; 511, 513; 521, 523), if said plunger (685, 785) is in said closed position.

2. The squeeze valve of claim 1, wherein said membrane (537) has a swelling (607) extending into said valve cavity (502; 512; 522; 602); the part of the swelling (607) extending into said valve cavity (502; 512; 522; 602) having a cross-section similar to the cross-section of the valve cavity (502; 512; 522; 602) in a plane perpendicular to the membrane.

3. A hose system for an injector for injecting contrast agents and saline solution into the human body comprising:
a squeeze valve of claims 1 or 2;
a contrast agent conduit (106, 126; 206, 226; 306, 326; 501, 502, 503, 504, 521, 522, 523, 524) for delivering a contrast agent;
a saline solution conduit (511, 512, 513, 514) for delivering a saline solution; said saline solution conduit (511, 512, 513, 515) comprising said valve cavity (512; 602) within said upper panel (535); said valve cavity (512; 602) may be closed by squeezing said membrane (537) into said valve cavity (512; 602).
a pump hose (132; 232; 332; 532) for delivering the contrast agent and the saline solution; said pump hose (132; 232; 332; 532) being fluidically connected to the contrast agent conduit (106, 126; 206, 226; 306, 326; 501, 502, 503, 504, 521, 522, 523, 524) and the saline solution conduit (116, 216, 316, 511, 512, 513, 515);
a frame (135; 235; 335; 535, 536, 537) to which said contrast agent conduit (106, 126; 206, 226; 306, 326; 501, 502, 503, 504, 521, 522, 523, 524) is mechanically fixed at a least one position; said saline solution conduit (116; 216; 316; 511, 512, 513, 514) being fixed to said frame (135; 235; 335; 535, 536, 537) at a least one position; said pump hose (132; 232; 332; 532) being fixed to said frame (135; 235; 335; 535, 536, 537) at at least two positions; said frame being comprised of said upper panel (535), a lower panel (536) and said membrane (537) being fixed between said upper panel (535) and said lower panel (536); said upper panel (535) being glued or welded to said lower panel (436; 536); said upper panel comprises guidances (501, 503, 511, 513, 521, 523) which form part of said contrast agent conduit or saline solution conduit; said frame comprising said upper panel (535) and said membrane (537) fixed to said upper panel (535); said upper panel (535) comprises guidances (501, 503, 511, 513, 521, 523, 531, 567, 568) said lower panel having a cut-out (625) below a guidance (567) within said upper panel (535).

4. The hose system of claim 3, said lower panel (536) comprising a recess (563, 564) around said cut-out (625); said recess (563, 564) constituting an abutment area (622) for a housing (623) of a pressure sensor (621); said membrane (537) comprising a swelling (577) which extends into said cut-out (625) such that said swelling (577) and said abutment area (622) form a flat surface if there is ambient pressure in said guidance (567).

5. The hose system of claim 3, wherein
said saline solution conduit (511, 512, 513, 515; 711) ends at an input of a saline solution valve (415; 512; 712);
said contrast agent conduit (501, 503, 504, 521, 523, 524) ends at an input of a contrast agent valve (502, 522; 702);
a first connection conduit (713) connects an output of said saline solution valve (512; 712) with an output of said contrast agent valve (502, 522; 702); and said pump hose (532) is fluidically connected (531; 731) to said output of said contrast agent valve (502, 522; 702).

6. The hose system of claim 5, wherein
the hose system comprises a second contrast agent conduit (521, 523, 524) which ends at an input of a second contrast agent valve (522; 722);
a second connection conduit (703) connects said output of said contrast agent valve (502; 702) with an output of said second contrast agent valve (522; 722); and
said pump hose (532) is fluidically connected (531; 731) to said output of said second contrast agent valve (522; 722).

7. The hose system of one of claims 3 to 6, wherein said pump hose (132; 232; 332; 432; 532) being fixed to said frame (135; 235; 335; 535, 536, 537) in a way that said pump hose (132; 232; 332; 532) forms a U or Ω between the two positions in which said pump hose (132; 232; 332; 532) is fixed to said frame (135; 235; 335; 535, 536, 537).

8. The hose system of one of the claims 3 to 7, wherein said frame (135; 235; 335; 535, 536, 537) comprises hook-like, tunnel-like or wall-like sections (8) for fixing hoses (106, 116, 126, 132; 206, 216, 226, 232; 306, 316, 326, 332; 532) to said frame (135; 235; 335; 535, 536, 537).

9. The hose system of one of the claims 3 to 8, wherein a contrast agent piercing needle (102, 122; 202, 222; 302, 322) is fluidically connected to said contrast agent conduit (106, 126; 206, 226; 306, 326; 501, 502, 503, 504, 521, 522, 523, 524) and a saline solution piercing needle (112; 212; 312) being connected to said saline solution conduit (116, 216, 316, 511, 512, 513, 515).

10. The hose system of claim 9, wherein said contrast agent piercing needle (202, 222) and said saline solution piercing needle (212) are fixed to said frame (235; 535, 536, 537).

11. The hose system of claim 9, wherein said hose system further comprises a second frame (336), to which said contrast agent piercing needle (302, 322) and said saline solution piercing needle (312) are fixed; at least part of said contrast agent conduit being formed by a flexible contrast agent hose (306, 326) and at least part of said saline solution conduit being formed by a flexible saline solution hose (316); said contrast agent hose (306, 326) and said saline solution hose (316) mechanically connect said frame (135; 235; 335; 535, 536, 537) and said second frame (336).

12. The hose system of one of the claims 3 to 11, wherein said frame (135; 235; 335; 535, 536, 537) constitutes part of the sterile packaging of said hose system for shipping said hose system.

13. The hose system of one of the claims 3 to 12, wherein said hose system further comprises an ultrasonic flow measuring section (551) before said pump hose (132; 232; 332; 532) in flow direction.

## Patentansprüche

1. Ein Quetschventil mit:
einer oberen Platte (535), die eine Ventilaussparung (502; 512; 522; 602) enthält, die zwei Teile einer Leitung (501, 503, 511, 513, 521, 523) abtrennt;
einer Membran (537), die an der oberen Platte (535) befestigt ist und die Ventilaussparung (502, 512, 522, 602) abdeckt und eine Seite der Teile der Leitung (501, 503, 511, 513, 521, 523) bildet; und
einem Presskolben (685, 785) mit einer offenen Position und einer geschlossenen Position, wobei der Presskolben (685, 785) die Membran in die Ventilaussparung (502, 512, 522, 602) hineinpresst und dabei die Teile der Leitung (501, 503, 511, 513, 521, 523) abtrennt, wenn sich der Presskolben (685, 785) in der geschlossenen Position befindet.

2. Das Quetschventil gemäß Anspruch 1, **dadurch gekennzeichnet, dass** die Membran (537) eine Verdickung (607) aufweist, die in die Ventilaussparung (502, 512, 522, 602) hineinragt, wobei der Teil der Verdickung (607), der in die Ventilaussparung (502, 512, 522, 602) hineinragt, in einer Ebene senkrecht zur Membran einen ähnlichen Querschnitt wie der Querschnitt der Ventilaussparung (502, 512, 522, 602) aufweist.

3. Schlauchsystem für einen Injektor zum injizieren von Kontrastmitteln und Kochsalzlösung in den menschlichen Körper mit:
einem Quetschventil gemäß Anspruch 1 oder 2;
einer Kontrastmittelleitung (106, 126, 206, 226, 306, 326, 501, 502, 503, 504, 521, 522, 523, 524) zum Fördern eines Kontrastmittels;
einer Kochsalzlösungsleitung (511, 512, 513, 514) zum Fördern einer Kochsalzlösung; wobei die Kochsalzlösungsleitung (511, 512, 513, 514) die Ventilaussparung (512, 602) im Inneren der oberen Platte (535) enthält, wobei die Ventilaussparung (512, 602) durch Quetschen der Membran (537) in diese Ventilaussparung (512, 602) geschlossen werden kann;
einem Pumpenschlauch (132, 232, 332, 532) zum Fördern des Kontrastmittels und der Kochsalzlösung, wobei der Pumpenschlauch (132, 232, 332, 532) strömungstechnisch an die Kontrastmittelleitung (106, 126, 206, 226, 306, 326, 501, 502, 503, 504, 521, 522, 523, 524) und an die Kochsalzlösungsleitung (116, 216, 316, 511, 512, 513, 515) angeschlossen ist;
einem Rahmen (135, 235, 335, 535, 536, 537), an dem die Kontrastmittelleitung (106, 126, 206, 226, 306, 326, 501, 502, 503, 504, 521, 522, 523, 524) an mindestens einer Stelle mechanisch befestigt ist, wobei die Kochsalzlösungsleitung (116, 216, 316, 511, 512, 513, 514) an mindestens einer Stelle am Rahmen (135, 235, 335, 535, 536, 537) befestigt ist; wobei der Pumpenschlauch (132, 232, 332, 532) am Rahmen (135, 235, 335, 535, 536, 537) an mindestens zwei Stellen befestigt ist, wobei der Rahmen die obere Platte (535), eine unteren Platte (536) und die Membran (537), die zwischen der unteren Platte (535) und der oberen Platte (536) befestigt ist, umfasst, wobei die obere Platte (535) an die unteren Platte (436, 536) angeklebt oder angeschweißt ist, wobei die obere Platte Führungen (501, 503, 511, 513, 521, 523) enthält, die einen Teil der Kontrastmittelleitung oder Kochsalzlösungsleitung bilden; wobei der Rahmen die obere Platte (535) und die Membran (537) enthält, die an der oberen Platte (535) befestigt ist; wobei die obere Platte (535) Führungen (501, 503, 511, 513, 521, 523, 531, 567, 568) enthält, wobei die untere Platte eine Aussparung (625) unter einer Führung (567) in der oberen Platte (535) enthält.

4. Schlauchsystem gemäß Anspruch 3, **dadurch gekennzeichnet, dass** die untere Platte (536) eine Eintiefung (563, 564) um die Aussparung (625) enthält, wobei die Eintiefung (563, 564) einen Widerlagerbereich (622) für ein Gehäuse (623) eines Drucksensors (621) bildet, wobei die Membran (537) eine Verdickung (577) aufweist, die in die Aussparung (625) hineinragt, so dass die Verdickung (577) und der Widerlagerbereich (622) eine flache Oberfläche bilden, wenn in der Führung (567) Umgebungsdruck herrscht.

5. Schlauchsystem gemäß Anspruch 3, **dadurch gekennzeichnet, dass**
die Kochsalzlösungsleitung (511, 512, 513, 515, 711) an einem Eingang eines Kochsalzlösungsventils (415, 512, 712) endet;
die Kontrastmittelleitung (501, 503, 504, 521, 523, 524) an einem Eingang eines Kontrastmittelventils (502, 522, 702) endet;
ein erster Verbindungskanal (713) einen Ausgang des Kochsalzlösungsventils (512, 712) mit einem Ausgang des Kontrastmittelventils (502, 522, 702) verbindet; und
der Pumpenschlauch (532) strömungstechnisch (531, 731) mit dem Ausgang des Kontrastmittelventils (502, 522, 702) verbunden ist.

6. Schlauchsystem gemäß Anspruch 5, **dadurch gekennzeichnet, dass**
das Schlauchsystem eine zweite Kontrastmittelleitung (521, 523, 524) enthält, die an einem Eingang eines zweiten Kontrastmittelventils (522, 722) endet;
ein zweiter Verbindungskanal (703) den Ausgang des Kontrastmittelventils (502, 702) mit einem Ausgang des zweiten Kontrastmittelventils (522, 722) verbindet; und
der Pumpenschlauch (532) strömungstechnisch (531, 731) mit dem Ausgang des zweiten Kontrastmittelventils (522, 722) verbunden ist.

7. Schlauchsystem gemäß einem der Ansprüche 3 bis 6, **dadurch gekennzeichnet, dass** der Pumpenschlauch (132, 232, 332, 432, 532) am Rahmen (135, 235, 335, 535, 536, 537) in einer Weise befestigt ist, dass der Pumpenschlauch (132, 232, 332, 532) zwischen den beiden Stellen, an denen der Pumpenschlauch (132, 232, 332, 532) am Rahmen (135, 235, 335, 535, 536, 537) befestigt ist, ein U oder Ω bildet.

8. Schlauchsystem gemäß einem der Ansprüche 3 bis 7, **dadurch gekennzeichnet, dass** der Rahmen (135, 235, 335, 535, 536, 537) hakenähnliche, tunnelähnliche und wandähnliche Abschnitte (8) enthält, um Schläuche (106, 116, 126, 132, 206, 216, 226, 232, 306, 316, 326, 332, 532) am Rahmen (135, 235, 335, 535, 536, 537) zu befestigen.

9. Schlauchsystem gemäß einem Ansprüche 3 bis 8, **dadurch gekennzeichnet, dass** ein Kontrastmitteleinstechdorn (102, 122, 202, 222, 302, 322) strömungstechnisch mit der Kontrastmittelleitung (106, 126, 206, 226, 306, 326, 501, 502, 503, 504, 521, 522, 523, 524) verbunden ist, und ein Kochsalzlösungseinstechdorn (112, 212, 312) mit der Kochsalzlösungsleitung (116, 216, 316, 511, 512, 513, 515) verbunden ist.

10. Schlauchsystem gemäß Anspruch 9, **dadurch gekennzeichnet, dass** der Kontrastmitteleinstechdorn (202, 222) und der Kochsalzlösungseinstechdorn (212) am Rahmen (235, 535, 536, 537) befestigt sind.

11. Schlauchsystem gemäß Anspruch 9, **dadurch gekennzeichnet, dass** das Schlauchsystem weiter einen zweiten Rahmen (336) enthält, an dem der Kontrastmitteleinstechdorn (302, 322) und der Kochsalzlösungseinstechdorn (312) befestigt sind, wobei mindestens ein Teil der Kontrastmittelleitung durch einen beweglichen Kontrastmittelschlauch (306, 326) gebildet wird und mindestens ein Teil der Kochsalzlösungsleitung durch einen beweglichen Kochsalzlösungsschlauch (316) gebildet wird, wobei der Kontrastmittelschlauch (306, 326) und der Kochsalzlösungsschlauch (316) den Rahmen (135, 235, 335, 535, 536, 537) mechanisch mit dem zweiten Rahmen (336) verbinden.

12. Schlauchsystem gemäß einem Ansprüche 3 bis 11, **dadurch gekennzeichnet, dass** der Rahmen (135, 235, 335, 535, 536, 537) einen Teil der sterilen Verpackung des Schlauchsystems zum Transportieren des Schlauchsystems bildet.

13. Schlauchsystem gemäß einem der Ansprüche 3 bis 12, **dadurch gekennzeichnet, dass** das Schlauchsystem weiter in Fliessrichtung vor dem Pumpenschlauch (132, 232, 332, 532) eine Ultraschallflussmessstrecke (551) enthält.

## Revendications

1. Soupape de compression comprenant :
un panneau supérieur (535) comprenant une cavité de soupape (502 ; 512 ; 522 ; 602) séparant deux parties d'un conduit (501, 503 ; 511, 513 ; 521, 523) ;
une membrane (537) fixée audit panneau supérieur (535) recouvrant ladite cavité de soupape (502 ; 512 ; 522 ; 602) et formant un côté desdites parties dudit conduit (501, 503 ; 511, 513 ; 521, 523) ; et
un piston (685, 785) ayant une position ouverte et une position fermée, ledit piston (685, 785) comprimant ladite membrane dans ladite cavité de soupape (502 ; 512 ; 522 ; 602), séparant ainsi lesdites parties dudit conduit (501, 503 ; 511, 513 ; 521, 523) lorsque ledit piston (685, 785) est dans ladite position fermée.

2. Soupape de compression selon la revendication 1, dans laquelle ladite membrane (537) a un renflement (607) s'étendant dans ladite cavité de soupape (502 ; 512 ; 522 ; 602) ; la partie du renflement (607) s'étendant dans ladite cavité de soupape (502 ; 512 ; 522 ; 602) ayant une section transversale similaire à la section transversale de la cavité de soupape (502 ; 512 ; 522 ; 602) dans un plan perpendiculaire à la membrane.

3. Système de tuyau flexible pour un injecteur servant à injecter des agents de contraste et une solution saline dans le corps humain, comprenant :
une soupape de compression selon les revendications 1 ou 2 ;
un conduit d'agent de contraste (106, 126 ; 206, 226 ; 306, 326 ; 501, 502, 503, 504, 521, 522, 523, 524) servant à administrer un agent de contraste ;
un conduit de solution saline (511, 512, 513, 514) servant à administrer une solution saline ; ledit conduit de solution saline (511, 512, 513, 515) comprenant ladite cavité de soupape (512 ; 602) prévue à l'intérieur dudit panneau supérieur (535) ; ladite cavité de soupape (512 ; 602) pouvant être fermée en comprimant ladite membrane (537) dans ladite cavité de soupape (512 ; 602).
un tuyau flexible de pompe (132 ; 232 ; 332 ; 532) servant à administrer l'agent de contraste et la solution saline ; ledit tuyau flexible de pompe (132 ; 232 ; 332 ; 532) étant relié sur le plan fluide au conduit d'agent de contraste (106, 126 ; 206, 226 ; 306, 326 ; 501, 502, 503, 504, 521, 522, 523, 524) et au conduit de solution saline (116, 216, 316, 511, 512, 513, 515) ;
un cadre (135 ; 235 ; 335 ; 535, 536, 537) auquel ledit conduit d'agent de contraste (106, 126 ; 206, 226 ; 306, 326 ; 501, 502, 503, 504, 521, 522, 523, 524) est fixé mécaniquement en au moins une position ; ledit conduit de solution saline (116 ; 216 ; 316 ; 511, 512, 513, 514) étant fixé audit cadre (135 ; 235 ; 335 ; 535, 536, 537) en au moins une position ; ledit tuyau flexible de pompe (132 ; 232 ; 332 ; 532) étant fixé audit cadre (135 ; 235 ; 335 ; 535, 536, 537) en au moins deux positions ; ledit cadre étant composé dudit panneau supérieur (535), d'un panneau inférieur (536) et de ladite membrane (537) fixée entre ledit panneau supérieur (535) et ledit panneau inférieur (536) ; ledit panneau supérieur (535) étant collé ou soudé audit panneau inférieur (436 ; 536) ; ledit panneau supérieur comprenant des éléments de guidage (501, 503, 511, 513, 521, 523) faisant partie dudit conduit d'agent de contraste ou dudit conduit de solution saline ; ledit cadre comprenant ledit panneau supérieur (535) et ladite membrane (537) fixée audit panneau supérieur (535) ; ledit panneau supérieur (535) comprenant les éléments de guidage (501, 503, 511, 513, 521, 523, 531, 567, 568), ledit panneau inférieur ayant un détouré (625) placé en dessous d'un élément de guidage (567), à l'intérieur dudit panneau supérieur (535).

4. Système de tuyau flexible selon la revendication 3, ledit panneau inférieur (536) comprenant un renfoncement (563, 564) autour dudit détouré (625) ; ledit renfoncement (563, 564) constituant une zone de butée (622) pour un carter (623) d'un capteur de pression (621) ; ladite membrane (537) comprenant un renflement (577) s'étendant dans ledit détouré (625) de telle sorte que ledit renflement (577) et ladite zone de butée (622) forment une surface plate lorsqu'une pression ambiante s'exerce dans ledit élément de guidage (567).

5. Système de tuyau flexible selon la revendication 3, dans lequel :
ledit conduit de solution saline (511, 512, 513, 515 ; 711) prend fin au niveau d'une entrée d'une soupape de solution saline (415 ; 512 ; 712) ;
ledit conduit d'agent de contraste (501, 503, 504, 521, 523, 524) prend fin au niveau d'une entrée d'une soupape d'agent de contraste (502, 522 ; 702) ;
un premier conduit de connexion (713) relie une sortie de ladite soupape de solution saline (512 ; 712) à une sortie de ladite soupape d'agent de contraste (502, 522 ; 702) ; et
ledit tuyau flexible de pompe (532) est relié sur le plan fluide (531 ; 731) à ladite sortie de ladite soupape d'agent de contraste (502, 522 ; 702).

6. Système de tuyau flexible selon la revendication 5, dans lequel :
le système de tuyau flexible comprend un second conduit d'agent de contraste (521, 523, 524) qui prend fin au niveau d'une entrée d'une seconde soupape d'agent de contraste (522 ; 722) ;
un second conduit de connexion (703) relie ladite sortie de ladite soupape d'agent de contraste (502 ; 702) à une sortie de ladite seconde soupape d'agent de contraste (522 ; 722) ; et
ledit tuyau flexible de pompe (532) est relié sur le plan fluide (531 ; 731) à ladite sortie de ladite seconde soupape d'agent de contraste (522 ; 722).

7. Système de tuyau flexible selon l'une quelconque des revendications 3 à 6, dans lequel ledit tuyau flexible de pompe (132 ; 232 ; 332 ; 432 ; 532) est fixé audit cadre (135 ; 235 ; 335 ; 535, 536, 537) de façon à ce que ledit tuyau flexible de pompe (132 ; 232 ; 332 ; 532) forme un U ou un Ω entre les deux positions dans lesquelles ledit tuyau flexible de pompe (132 ; 232 ; 332 ; 532) est fixé audit cadre (135 ; 235 ; 335 ; 535, 536, 537).

8. Système de tuyau flexible selon l'une quelconque des revendications 3 à 7, dans lequel ledit cadre (135 ; 235 ; 335 ; 535, 536, 537) comprend des sections (8) en forme de crochet, de tunnel ou de paroi pour fixer les tuyaux flexibles (106, 116, 126, 132 ; 206, 216, 226, 232 ; 306, 316, 326, 332 ; 532) audit cadre (135 ; 235 ; 335 ; 535, 536, 537).

9. Système de tuyau flexible selon l'une quelconque des revendications 3 à 8, dans lequel une aiguille de perçage d'agent de contraste (102, 122 ; 202, 222 ; 302, 322) est reliée sur le plan fluide audit conduit d'agent de contraste (106, 126 ; 206, 226 ; 306, 326 ; 501, 502, 503, 504, 521, 522, 523, 524) et une aiguille de perçage de solution saline (112 ; 212 ; 312) est reliée audit conduit de solution saline (116, 216, 316, 511, 512, 513, 515).

10. Système de tuyau flexible selon la revendication 9, dans lequel ladite aiguille de perçage d'agent de contraste (202, 222) et ladite aiguille de perçage de solution saline (212) sont fixées audit cadre (235 ; 535, 536, 537).

11. Système de tuyau flexible selon la revendication 9, dans lequel ledit système de tuyau flexible comprend en outre un second cadre (336) auquel ladite aiguille de perçage d'agent de contraste (302, 322) et ladite aiguille de perçage de solution saline (312) sont fixées ; au moins une partie dudit conduit d'agent de contraste étant formée par un tuyau flexible d'agent de contraste (306, 326) et au moins une partie dudit conduit de solution saline étant formé par un tuyau flexible de solution saline (316) ; ledit tuyau flexible d'agent de contraste (306, 326) et ledit tuyau flexible de solution saline (316) reliant mécaniquement ledit cadre (135 ; 235 ; 335 ; 535, 536, 537) et ledit second cadre (336).

12. Système de tuyau flexible selon l'une quelconque des revendications 3 à 11, dans lequel ledit cadre (135 ; 235 ; 335 ; 535, 536, 537) fait partie de l'emballage stérile dudit système de tuyau flexible pour l'expédition dudit système de tuyau flexible.

13. Système de tuyau flexible selon l'une quelconque des revendications 3 à 12, dans lequel ledit système de tuyau flexible comprend en outre une section de mesure du débit ultrasonique (551) avant ledit tuyau flexible de pompe (132 ; 232 ; 332 ; 532) dans la direction d'écoulement.
